# EUROPEAN PATENT APPLICATION

(11) **EP 4 574 071 A2**
(43) Date of publication of application: **25.06.2025**
(21) Application number: 24220433.7
(22) Date of filing: 17.12.2024
(51) Int. Cl.: A61B 18/14, A61B 18/00, A61B 5/00

(54) **SIMPLIFIED BASKET CATHETERS HAVING MULTIPLE SPINES**

(30) Priority: 18.12.2023 US 202318544059
(71) Applicant: Biosense Webster (Israel) Ltd., 2066717 Yokneam (IL)
(72) Inventor: JIMENEZ, Jose, Irvine, 92618 (US); PATEL, Amar, Irvine, 92618 (US); SUAREZ, Paul, Irvine, 92618 (US); NGUYEN, Thanh, Irvine, 92618 (US); RORICK, Kevin, Irvine, 92618 (US); XU, Guo, Irvine, 92618 (US); ROSEBERRY, Jacob, Irvine, 92618 (US)
(74) Representative: Carpmaels & Ransford LLP

(57) **Abstract**

A medical probe can comprise a tube comprising a proximal end and a distal end, the tube extending along a longitudinal axis. The medical probe can further comprise a plurality of spines disposed at the distal end of the tube, each spine of the plurality of spines comprising a first end that is fixed in relation to the tube and a second end that is unattached in relation to the tube. Each spine of the plurality of spines can be configured to transition between a collapsed configuration and an expanded configuration, the plurality of spines forming a basket when in the expanded configuration. The medical probe can further comprise a plurality of electrodes attached to the plurality of spines.

## Description

### FIELD

The present invention relates generally to medical devices, and in particular, simplified designs for basket catheters having a plurality of individually assembled spines.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. Electrical signals propagated through the heart can be mapped using a mapping catheter and then only selected areas of the tissue can be ablated to treat the AF. By selectively ablating cardiac tissue by application of energy via a catheter, it is sometimes possible to cease or modify the propagation of unwanted electrical signals from one portion of the heart to another. Medical probes may utilize radiofrequency (RF) electrical energy to heat tissue. Some ablation approaches use irreversible electroporation (IRE) to ablate cardiac tissue using nonthermal ablation methods.

To perform the operations of delivering ablative energy and mapping electrical signals propagated through cardiac tissue, physicians may use the same or a different catheter respectively. However, it is most beneficial to use a catheter capable of performing both and even additional functions. In some examples, a basket catheter with a plurality of electrodes disposed along the spines may be used, the electrodes capable of delivering ablative energy as well as mapping procedures. However, due to the small size of the spines and the electrodes, manufacturing such catheters may be difficult and/or expensive. What is needed, therefore, are systems and methods of manufacturing basket catheters in a cost-effective method with a simplified assembly. This and other issues can be addressed by the technology disclosed herein.

### SUMMARY

The disclosed technology includes a medical probe. The medical probe can comprise a tube comprising a proximal end and a distal end, the tube extending along a longitudinal axis. The medical probe can further comprise a plurality of spines disposed at the distal end of the tube, each spine of the plurality of spines comprising a first end that is fixed in relation to the tube and a second end that is unattached in relation to the tube. Each spine of the plurality of spines can be configured to transition between a collapsed configuration and an expanded configuration, the plurality of spines forming a basket when in the expanded configuration. The medical probe can further comprise a plurality of electrodes attached to the plurality of spines.

The disclosed technology can include a medical probe. The medical probe can comprise a tube comprising a proximal end and a distal end, the tube extending along a longitudinal axis. The medical probe can further comprise a plurality of spines disposed at the distal end of the tube, each spine of the plurality of spines comprising a first end and a second end, each being fixed in relation to the tube. Each spine of the plurality of spines can be configured to transition between a collapsed configuration and an expanded configuration. The plurality of spines can form a basket when in the expanded configuration by folding approximately at a midpoint of each spine and bowing radially outward from the longitudinal axis. The medical probe can further comprise a plurality of electrodes attached to the plurality of spines.

The disclosed technology can include a medical probe. The medical probe can comprise a tube comprising a proximal end and a distal end, the tube extending along a longitudinal axis. The medical probe can further comprise a single spine disposed at a distal end of the tube and comprising a first end and a second end, each end fixed in relation to the tube. The spine can be configured to transition between a collapsed configuration and an expanded configuration. The spine can form a basket comprising multiple lobes when in the expanded configuration by forming a plurality of bends, the multiple lobes bowing radially outward from the longitudinal axis. The medical probe can further comprise a plurality of electrodes attached to the spine.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is a schematic pictorial illustration of a medical system including a medical probe having an end effector with electrodes, in accordance with an embodiment of the disclosed technology;
FIG. 2 is a schematic pictorial illustration of a medical probe comprising an end effector, in accordance with the disclosed technology;
FIG. 3A is a front perspective view of an end effector comprising a plurality of spines separated by spacers with the end effector shown in the collapsed configuration, in accordance with the disclosed technology;
FIG. 3B is a front perspective view of the end effector of FIG. 3A with the spines shown as transitioning from the collapsed configuration to the expanded configuration, in accordance with the disclosed technology;
FIG. 3C is a front perspective view of the end effector of FIG. 3A fully in the expanded configuration, in accordance with the disclosed technology;
FIG. 4A is a front perspective view of an end effector comprising a plurality of spines bowing inwardly, the end effector being shown in the collapsed configuration, in accordance with the disclosed technology;
FIG. 4B is a front perspective view of the end effector of FIG. 4A transitioning from the collapsed configuration to the expanded configuration, in accordance with the disclosed technology;
FIG. 4C is a front perspective view of the end effector of FIG. 4A fully in the expanded configuration, in accordance with the disclosed technology;
FIG. 5A is a front perspective view of an end effector comprising a plurality of spines bowing outwardly, the end effector being shown in the collapsed configuration, in accordance with the disclosed technology;
FIG. 5B is a front perspective view of the end effector of FIG. 5A transitioning from the collapsed configuration to the expanded configuration, in accordance with the disclosed technology;
FIG. 5C is a front perspective view of the end effector of FIG. 5A fully in the expanded configuration, in accordance with the disclosed technology;
FIG. 6A is a front perspective view of an end effector comprising a plurality of spines, in accordance with the disclosed technology;
FIG. 6B is a top view of the distal end of the end effector of FIG. 6A, in accordance with the disclosed technology;
FIG. 7A is a front perspective view of an end effector comprising a single spine and in the collapsed configuration, in accordance with the disclosed technology;
FIG. 7B is a front perspective view of the end effector of FIG. 7A transitioning from the collapsed configuration to the expanded configuration, in accordance with the disclosed technology;
FIG. 7C is a front perspective view of the end effector of FIG. 7A fully in the expanded configuration, in accordance with the disclosed technology;
FIG. 8 is a flowchart illustrating a method of manufacture for a medical probe, in accordance with the disclosed technology.

### DETAILED DESCRIPTION

The disclosed technology includes a plurality of individual spines connected to an insertion tube and configured to form a basket shape when disposed from the insertion tube. The disclosed technology can simplify the process of manufacturing a basket catheter by including spines that are heat-set to turn into a pre-determined shape. Further, the disclosed technology can help reduce the amount of individual components required to manufacture such catheters, thereby reducing the overall complexity and cost of the basket catheter. The disclosed medical device can be configured to perform multiple procedures and functions through the use of one device.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives, and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "physician" or "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

The disclosed technology can be configured to deliver monophasic or biphasic pulses to ablate tissue. For example, the electrodes described herein configured to deliver ablative energy to tissue can be configured to deliver monophasic pulses, biphasic pulses, or some combination thereof. The terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

Example systems, methods, and devices of the present disclosure may be particularly suited for IRE ablation of cardiac tissue to treat cardiac arrhythmias. Ablative energies are typically provided to cardiac tissue by electrodes which can deliver ablative energy alongside the tissue to be ablated. Ablative procedures incorporating such example catheters can be visualized using fluoroscopy, magnetic-based position sensing, and/or active current location techniques.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

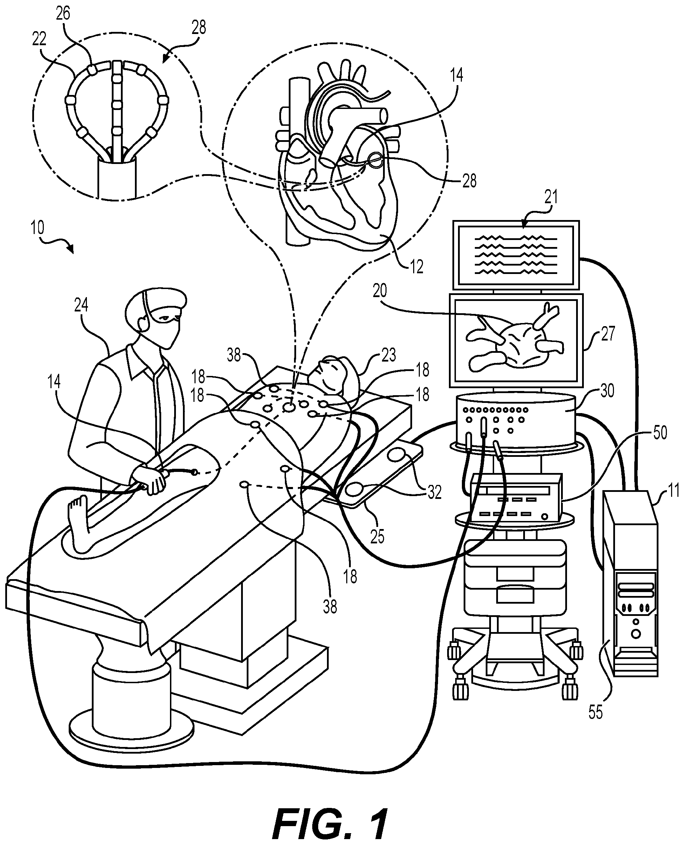

Reference is made to FIG. 1 showing an example catheter-based electrophysiology mapping and ablation system 10. System 10 includes one or more catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The one or more catheters may include catheters dedicated for sensing Intracardiac Electrogram (IEGM) signals, catheters dedicated for ablating and/or catheters dedicated for both sensing and ablating. An example catheter 14 (also referred to as medical probe 100 here) that is configured for sensing IEGM is illustrated herein. For ablation, physician 24 brings end effector 28 comprising ablation electrodes to a target site for ablating. If the end effector 28 is alternatively or additionally configured for mapping of electrophysiological signals (e.g., IEGM signals), physician 24 similarly brings the end effector 28 into contact with the heart wall for sensing a target site in heart 12.

Catheter 14 is an exemplary catheter that includes an end effector 28 comprising one and preferably multiple electrodes 26 optionally distributed over an expandable assembly and a distal tip of end effector 28 and configured to detect electro-physiological signals and/or deliver ablative energy to tissue. Catheter 14 may additionally include a magnetic-based position sensor embedded in or near end effector 28 for tracking position and orientation of end effector 28. The end effector 28 can further include one or more impedance-based electrodes disposed in or near end effector 28 for tracking position and orientation of end effector 28.

Magnetic-based position sensor may be operated together with a location pad 25 including a plurality of magnetic coils 32 configured to generate magnetic fields in a predefined working volume. Real time position of end effector 28 of catheter 14 may be tracked based on magnetic fields generated with location pad 25 and sensed by magnetic-based position sensor 29. The magnetic-based position sensor can be a single axis sensor, a dual axis sensor, or a triple axis sensor depending on the particular configuration. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; and 6,892,091, each of which is incorporated herein by reference as if set forth fully herein.

System 10 includes one or more electrode patches 38 positioned for skin contact on patient 23 to establish location reference for location pad 25 as well as tracking of impedance-based electrodes. For impedance-based tracking, electrical current is directed toward impedance-based electrodes and sensed at electrode skin patches 38 so that the location of each electrode can be triangulated via the electrode patches 38. Details of the impedance-based location tracking technology are described in U.S. Patent Nos. 7,536,218; 7,756,576; 7,848,787; 7,869,865; and 8,456,182, each of which is incorporated herein by reference as if set forth fully herein.

A recorder 11 displays electrograms 21 captured with body surface ECG electrodes 18 and intracardiac electrograms (IEGM) captured with electrodes of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes disposed on the end effector and configured for delivering ablative energy to tissue. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

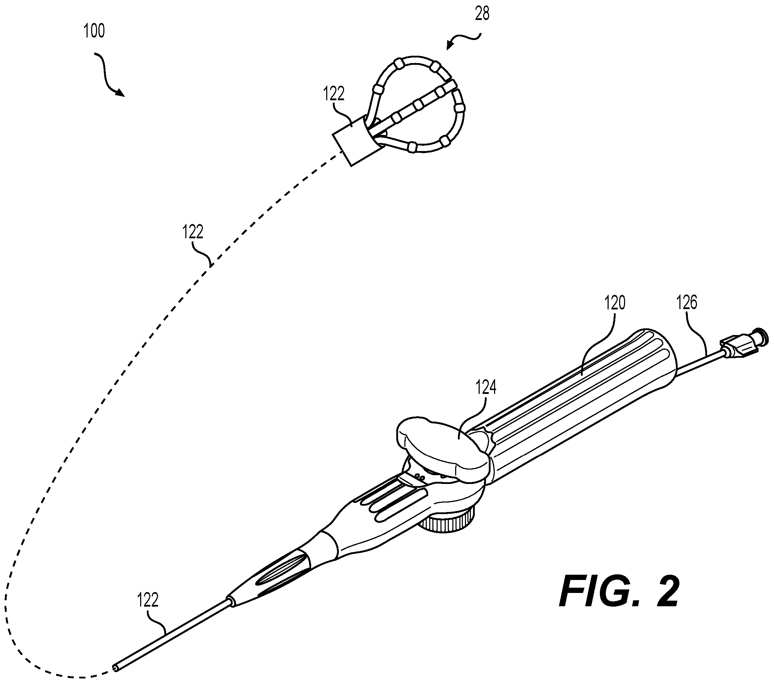

FIG. 2 illustrates an example medical probe 100 configured for insertion into an organ (e.g., heart 12) of a patient 23. As shown, the medical probe can comprise a handle 120, the handle 120 can include an irrigation port 126, an actuator 124, and be connected to a tube 122. The irrigation port 126 can be configured to connect to, and receive irrigation fluid from, an irrigation supply to deliver irrigation fluid to the end effector 28. The actuator 124 can be connected to a pull wire that is attached near the distal end of the tube 122 or the end effector 28. When the actuator 124 is actuated, the pull wire can be pulled to cause the distal end of the tube 122 to deflect away from the longitudinal axis 150.

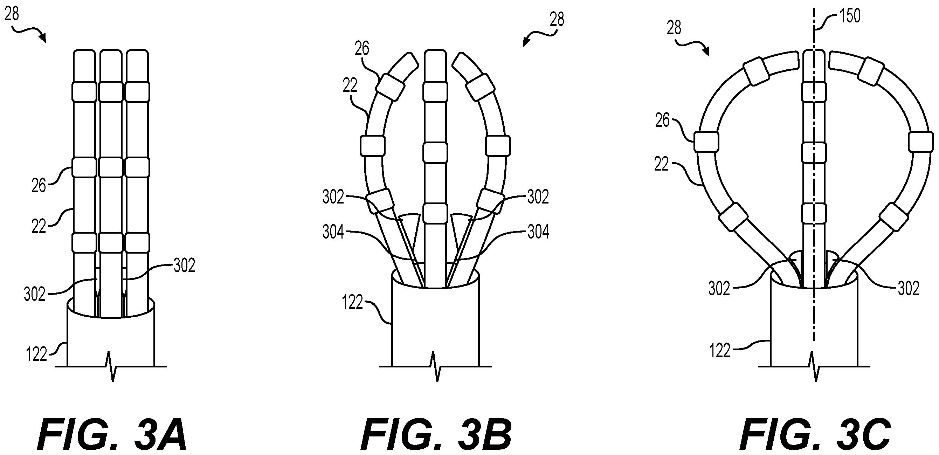

An end effector 28 can be attached to a distal end of the tube 122 and the end effector 28 can comprise a plurality of spines 22 that can be configured to bow radially outward from a longitudinal axis 150 (e.g., as shown in FIG. 3C) of the end effector 28. Each spine 22 can be configured to transition from a collapsed configuration to an expanded configuration when pushed out of an insertion sheath or other delivery tube. The spines 22 can each be configured to transition to the expanded configuration by turning inwardly at a distal end and bowing radially outward from the longitudinal axis 150 of the end effector 28, thereby forming a basket catheter.

Each spine 22 of the plurality of spines 22 can include a first end and a second end such that the first end is attached to the tube 122 and the second end is unattached to the tube 122 (e.g., the second end can be a free end). Furthermore, each spine 22 of the plurality of spines 22 can include a plurality of electrodes 26 disposed along each spine 22 with the plurality of electrodes 26 extending from the first end to the second end of the spine 22. In some examples, each spine 22 of the plurality of spines 22 can be made from a shape-memory material. The shape-memory material, for example, can be nitinol or other biocompatible materials that can be biased to form a basket shape when deployed from an insertion sheath. For example, the spines 22 can comprise nitinol and the nitinol can be heat-set to a predetermined shape (e.g., the deployed configuration) such that when the spines 22 are inserted into a blood pool at a predetermined temperature, the spines 22 will transition to the heat-set deployed configuration. In some examples, each spine 22 of the plurality of spines 22 can be covered in a layer of biocompatible insulative material.

The electrodes 26 can be made from a biocompatible conductive material (e.g., gold, palladium, silver, platinum) and be configured to deliver ablative energy to tissue. For example, the electrodes 26 can each be in electrical communication with the ablation energy generator 50 and configured to deliver the ablation energy to tissue. The electrodes 26 can be configured to deliver unipolar or bipolar signals with monophasic or biphasic pulses. Alternatively, or in addition, the electrodes 26 can be configured to deliver RF ablation energy to the tissue. Alternatively, or in addition, the electrodes 26 can be configured for performing mapping of electro-anatomical signals. For instance, the electrodes 26 described herein can be configured to detect electro-anatomical signals for a mapping procedure, for delivering ablative energy to tissue, or both. In some examples, the medical probe can further include a position sensor (not shown) configured to generate a current when subjected to an electromagnetic field.

FIGS. 3A-3C illustrate an example end effector 28 with spines 22 transitioning from a collapsed configuration (FIG. 3A) to an expanded configuration (FIG. 3C), the end effector 28 defining a distal end of the tube 122. The end effector 28 is substantially similar to the end effector 28 illustrated in FIG. 2 and can further comprise spacers 302 disposed between spines 22 of the plurality of spines 22. The spacers 302 can be connected to pull wires 304 that can be pulled proximally to move the spacers 302 between the spines 22 near the distal end of the tube 122. The spacers 302 disposed between the spines 22 are configured to cause the spines 22 to be spaced apart from each other when the end effector 28 is in the expanded configuration and the puller wires 304 connected to the spacers 302 are pulled in a proximal direction. In some examples, the end effector 28 can comprise a single puller wire 304 connected to a spacer 302, or alternatively, can comprise multiple puller wires 304 connected to each spacer 302. Each spine 22 of the plurality of spines 22 can further include a plurality of electrodes 26 disposed on the spine 22 and extending from a first end (a proximal end) to a second end (a distal end) of the spine 22.

FIG. 3A illustrates the end effector 28 in the collapsed configuration wherein the end effector 28 is prepared for delivery and navigation through an organ (e.g., heart 12) of a patient 23. In the collapsed configuration, each spine 22 is fully elongated and the second end (the distal end in this case) of each spine 22 is the furthest distance possible along the spine 22 from the tube 122 of the medical probe. FIG. 3B illustrates the end effector 28 in a configuration that is transitioning between the collapsed (FIG. 3A) and expanded (FIG. 3C) configurations. As can be seen, the puller wires 304 attached to the spacers 302 between the plurality of spines 22 are partially pulled in a proximal direction in order to further separate the spines 22 as they transition into the expanded configuration. FIG. 3C illustrates the end effector 28 of the medical probe 100 now fully in the expanded configuration, thereby forming basket shape. In the expanded configuration, the puller wires 304 are fully pulled in the proximal direction, which in turn, fully separates the spines 22 near the first end of each spine 22.

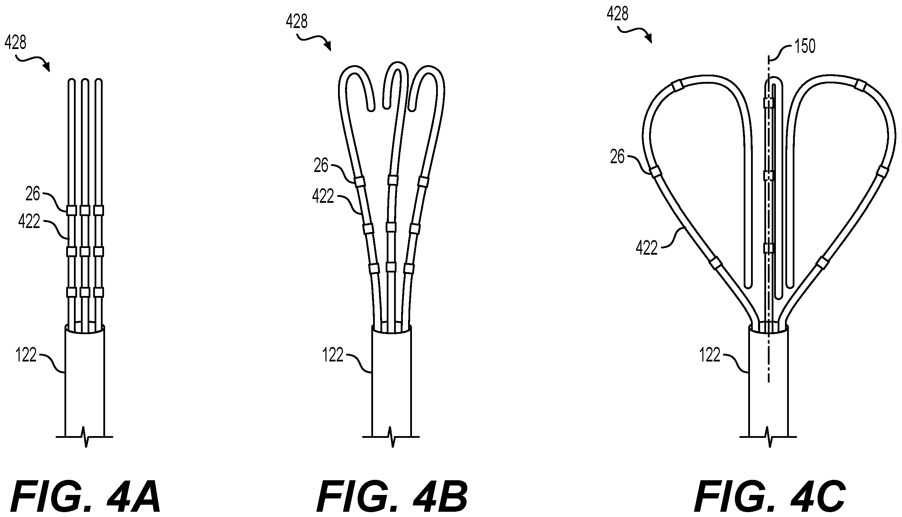

FIGS. 4A-4C illustrate an end effector 428 transitioning from a collapsed configuration (FIG. 4A) to an expanded configuration (FIG. 4C) and forming a basket catheter, the end effector 428 defining a distal end of the tube 122. The end effector 428 can comprise a plurality of spines 422, each spine 422 having a first end connected to the tube 122 and a second end unattached to the tube 122 (e.g., a free end). Each spine 422 of the plurality of spines 422 can include a plurality of electrodes 26 disposed along each spine 422. The electrodes 26 can be positioned along the spines 422 such that, when the end effector 428 is in the expanded configuration and forms a basket, the plurality of electrodes 26 extend from the first end of each spine 422 to a distal end of the basket. For example, as shown in FIG. 4A, the electrodes 26 can be disposed nearer a proximal end of each spine 422 such that when the distal end of the spine 422 rotates inwardly, the electrodes 26 are disposed on a portion of the spine 422 that is configured to contact tissue.

FIG. 4A illustrates the end effector 428 in the collapsed configuration wherein the end effector 428 is prepared for delivery and navigation through an organ (e.g., heart 12) of a patient 23. In the collapsed configuration, each spine 422 is fully elongated and the second end of each spine 422 is the portion of the spine 422 positioned most distal from the tube 122 of the medical probe 100. As can be seen, the plurality of electrodes 26 are disposed along a portion of the spine 422. FIG. 4B illustrates the end effector 428 in a configuration that is transitioning between the collapsed (FIG. 4A) and expanded (FIG. 4C) configurations. As shown in FIG. 4B, as each spine 422 begins to transition from the collapsed configuration to the expanded configuration, the second end of each spine 422 begins to turn inwardly toward the longitudinal axis 150 and a portion of the spine 422 near the second end begins to bow radially outward from the longitudinal axis 150.

FIG. 4C illustrates the end effector 428 of the medical probe now fully in the expanded configuration, thereby forming the basket shape. The plurality of spines 422 of the end effector 428 can be configured to turn the second end of each spine 422 inwardly toward the longitudinal axis 150 such that the second end is disposed near the first end of the spine 422. The portion of the spine 422 disposed between the first end and second end of the spine 422 can bow radially outward from the longitudinal axis 150, thereby forming the basket shape. As a result, a basket is formed which in turn causes the plurality of electrodes 26 disposed along the spines 422 to extend from the first end of the spine 422 to the distal end of the basket. This allows the plurality of electrodes 26 to be disposed on a portion of the basket catheter that will contact the target tissue and position the electrodes 26 for delivery of ablative energy to the tissue.

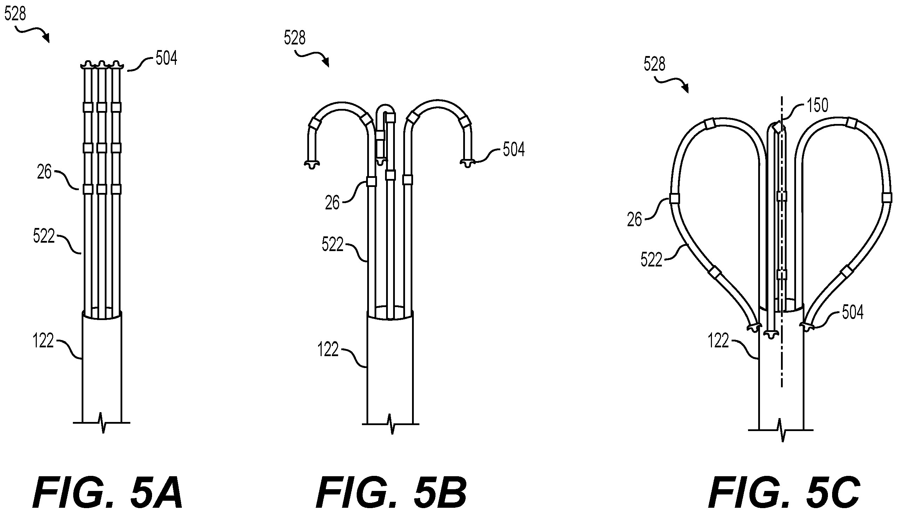

Turning now to FIGS. 5A-5C which illustrate an alternative end effector 528 transitioning from a collapsed configuration (FIG. 5A) to an expanded configuration (FIG. 5C). The end effector 428 can comprise a plurality of spines 522 with each spine 522 having a first end attached to the tube 122 and a second end unattached to the tube 122 (a free end). Each spine 522 of the plurality of spines 522 can further include a plurality of electrodes 26 disposed along each spine 522. Furthermore, when the end effector 528 is in the expanded configuration, the electrodes 26 can be positioned along the spines 522 such that the plurality of electrodes 26 extend from the second end to a distal end of the basket. For example, as shown in FIG. 5A, the electrodes 26 can be disposed nearer a distal end of each spine 522 such that when the distal end of the spine 22 rotates outwardly, the electrodes 26 are disposed on a portion of the spine 522 that is configured to contact tissue.

Each spine 522 of the plurality of spines 522 can further include an anchor 504 disposed at the second end of the spines 522. The anchors 504 can be configured to, when the end effector 528 is in the expanded configuration and forms a basket, attach to the tube 122 of the medical probe. For example, as the spines 522 bend outwardly and curl back toward the tube 122, the anchors 504 can attach to the tube 122 to secure the second ends of the spines 522.

FIG. 5A illustrates the end effector 528 in the collapsed configuration wherein the end effector 528 is prepared for delivery and navigation through an organ (e.g., heart 12) of a patient 23. In the collapsed configuration, each spine 522 is fully elongated and the second end of each spine 522 is the portion of the spine 522 positioned most distal from the tube 122 of the medical probe 100. As can be seen, the plurality of electrodes 26 are disposed along a portion of the spine 522 that is more distal the tube 122. FIG. 5B illustrates the end effector 428 in a configuration that is transitioning between the collapsed (FIG. 5A) and expanded (FIG. 5C) configurations. As shown in FIG. 5B, as the spine 522 begins to transition from the collapsed configuration to the expanded configuration, the second end of each spine 522 begins to turn outwardly from the longitudinal axis 150 and a portion of the spine 522 near the second end begins to turn inward back toward the tube 122.

FIG. 5C illustrates the end effector 528 of the medical probe 100 now in the expanded configuration, thereby forming the basket shape. The plurality of spines 522 of the end effector 528 can be configured to turn the second end of each spine 522 outwardly away from the longitudinal axis 150 such that the second end is disposed near the first end of the spine 522 and the anchor 504 is attached to the outside of the tube 122. In other examples, the anchors can be configured to, when the end effector 528 is in the expanded configuration, alternatively attach to the inside of the tube 122 (e.g., the spines 522 curl inwardly such that the second end of the spines 522 at least partially insert into an opening formed at a distal end of the tube 122). The portion of the spine 522 disposed between the first end and second end of the spine 522 can bow radially outward from the longitudinal axis 150, thereby forming the basket shape. As a result, a basket is formed which in turn causes the plurality of electrodes 26 disposed along the spines 522 to extend from the second end of the spine 522 to the distal end of the basket. This allows the plurality of electrodes 26 to be disposed on an area of the basket catheter that will contact the target tissue and allow them to deliver ablative energy and/or detect electrophysiological signals.

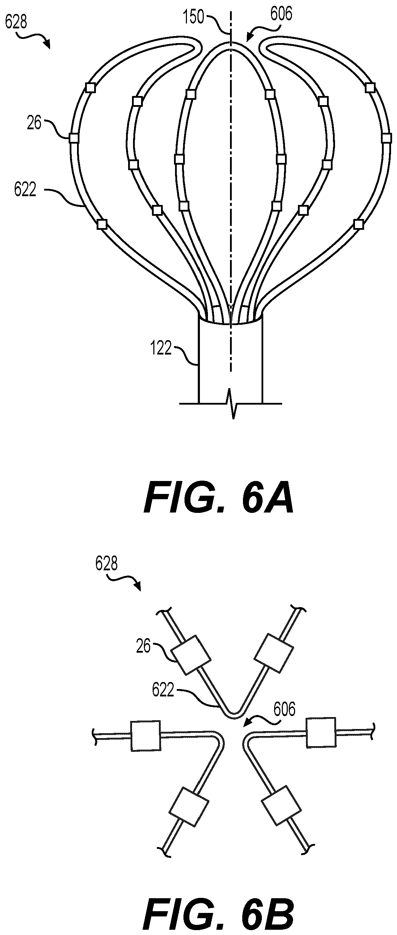

Now referencing FIGS. 6A-6B, which illustrate an alternative end effector 628 disposed at a distal end of tube 122. FIG. 6A illustrates a front view of the end effector 628 in the expanded configuration. Although the collapsed configuration is not shown, it will be appreciated that the spines 622 can be substantially straight when in the collapsed configuration for delivery through vasculature. The end effector 628 can comprise a plurality of spines 622, each spine 622 having a first end and a second end with each of the first end and the second end being connected to the tube 122. The plurality of spines 622 can include a plurality of electrodes 26 disposed along the spines 622 that extend from the first end to the second end.

FIG. 6B illustrates a top view of the spines 622 of the end effector 628 of FIG. 6A. Each spine 622 of the plurality of spines 622 can be configured to transition from a collapsed configuration to an expanded configuration by folding approximately at a midpoint of each spine 622 and bowing radially outward from the longitudinal axis 150. The midpoints of each spine 622 can be configured to converge towards the longitudinal axis 150 at a convergence point 606. As a result, a basket shape is formed having a distal end formed by the midpoints of each spine 622 converging toward each other at the convergence point 606. In FIGS. 6A-6B the plurality of spines 22 comprises 3 spines 622, however the end effector 628 can comprise four spines, five spines, six spines, ten spines, twenty spines, or any number of spines as suitable for the particular application.

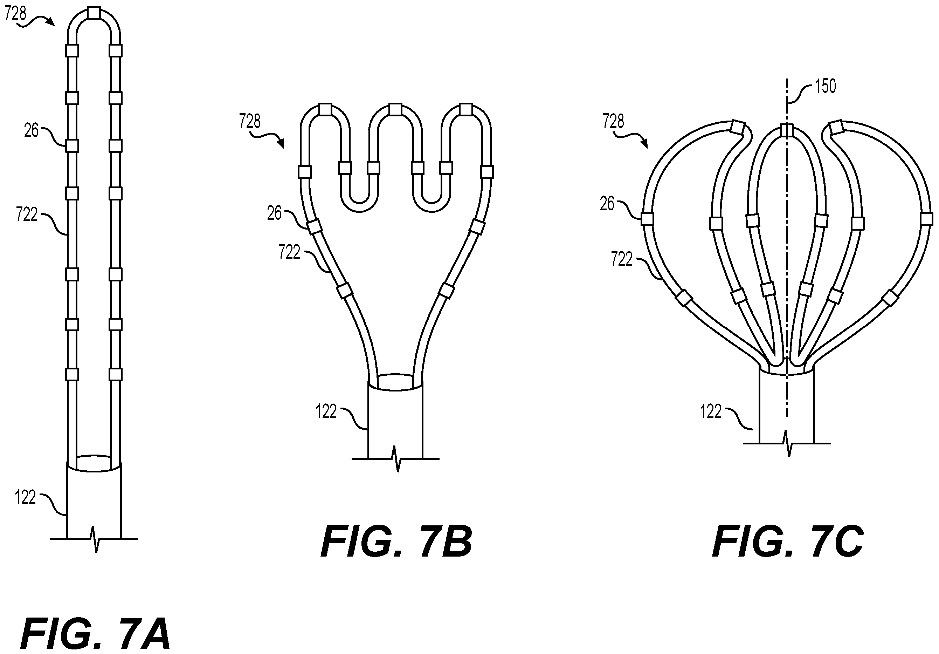

FIGS. 7A-7C illustrate an alternative embodiment of an end effector 728 that defines a distal end of the tube 122. Unlike the example end effectors previously described, the end effector 728 shown in FIGs. 7A-7C can comprise a single spine 722. The spine 722 can include a first end and a second end, each end connected to the tube 122. The spine 722 can be configured to transition between a collapsed configuration (FIG. 7A) and an expanded configuration (FIG. 7C) such that when in the expanded configuration the spine 722 forms a basket shape comprising multiple lobes. In FIGS. 7C the end effector 728 is shown as having three lobes, however an end effector can comprise four lobes, five lobes, six lobes, ten lobes, twenty lobes, or any other number of lobes as would be suitable for the particular application. The spine 722 can further include a plurality of electrodes 26 disposed along the spines 722, the plurality of electrodes 26 extending from the first end to the second end of the spine 722.

FIG. 7A illustrates the end effector 728 in the collapsed configuration wherein the end effector 728 is prepared for delivery and navigation through an organ (e.g., heart 12) of a patient 23. In the collapsed configuration the spine 722 of the end effector 728 is fully elongated and a portion of the spine 722 develops a single bend positioned furthest from the tube 122 of the medical probe. FIG. 7B illustrates the end effector 728 in a configuration that is transitioning between the collapsed (FIG. 7A) and expanded (FIG. 7C) configurations. The spine 722 can begin to form three lobes that comprises a plurality of bends in the spine 722. As shown in FIG. 7B, the lobes of the spine 722 can at least partially bow radially outward from the longitudinal axis 150 of the tube 122. FIG. 7C illustrates the end effector 728 in the expanded configuration wherein the lobes of the spine 722 fully bow radially outward from the longitudinal axis 150, thereby forming the basket shape. In the expanded configuration, the proximal-most bends of the plurality of bends converge towards the tube 122 and the distal-most bends of the plurality of bends converges toward a distal end of the basket.

Although not shown, it will be appreciated that the spines 22 described herein (22, 422, 522, 622, and/or 722) can include a notch, hole, or other features to allow the spines 22 to interlock with each other. This can be particularly helpful, for example, if the spines 22 interlock near a distal end of the basket to strengthen the basket shape to prevent distortion when the basket is brought into contact with tissue.

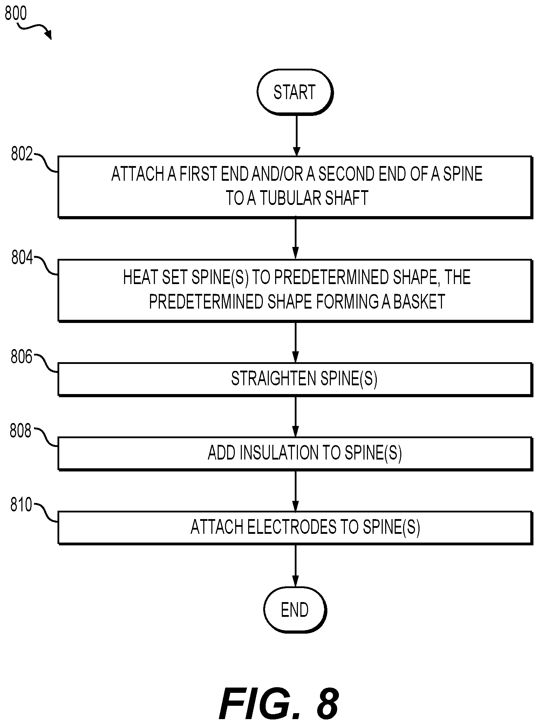

FIG. 8 is a flowchart illustrating a method 800 of manufacturing a medical probe. In some examples, the medical probe disclosed in method 800 is substantially similar to any of the medical probes illustrated and described in relation to FIGS. 3A-7C. Method 800 can include attaching 802 a first end and/or a second end of a spine 22 to a tube 122. The method 800 can further include heat setting 804 the spine(s) 22 to a pre-determined shape, the pre-determined shape forming a basket. In some examples, the basket can comprise a plurality of lobes that form multiple bends in the spine(s) 22. The method 800 can further include straightening 806 the spine(s) 22 and adding 808 insulation to the spine(s) 22. The method 800 can further include attaching 810 electrodes 26 to the spine(s) 22. As stated above, each electrode 26 can be configured to detect electrophysiological signals and/or deliver ablative anergy to target tissue of an organ (e.g., heart 12). In some examples, the medical probe can further include a position sensor (not shown) configured to generate a current when subjected to an electromagnetic field.

The disclosed technology described herein can be further understood according to the following clauses:
Clause 1: A medical probe comprising: a tube comprising a proximal end and a distal end, the tube extending along a longitudinal axis; a plurality of spines disposed at the distal end of the tube, each spine of the plurality of spines comprising a first end that is fixed in relation to the tube and a second end that is unattached in relation to the tube, each spine of the plurality of spines configured to transition between a collapsed configuration and an expanded configuration, the plurality of spines forming a basket when in the expanded configuration; and a plurality of electrodes attached to the plurality of spines.
Clause 2: The medical probe of clause 1, each spine of the plurality of spines configured to turn inwardly at a distal end and bow radially outward from the longitudinal axis when deployed from an insertion sheath.
Clause 3: The medical probe of clause 2 further comprising a spacer disposed between the plurality of spines so that the plurality of spines are spaced apart from each other.
Clause 4: The medical probe of clause 3, the spacer being connected to a pull wire, the pull wire being configured to cause the spacer to move proximally thereby causing the plurality of spines to be spaced apart from each other.
Clause 5: The medical probe of any of the preceding clauses, the plurality of electrodes being disposed along each spine of the plurality of spines from the first end to the second end.
Clause 6: The medical probe of any of the preceding clauses, the plurality of electrodes being configured to detect electrophysiological signals.
Clause 7: The medical probe of any of the preceding clauses, the plurality of electrodes being configured to deliver ablative energy to tissue.
Clause 8: The medical probe of clause 1, each spine of the plurality of spines configured to turn inwardly toward the longitudinal axis such that the second end is disposed near the first end and each spine bows radially outward from the longitudinal axis when deployed from an insertion sheath.
Clause 9: The medical probe of clause 8, the plurality of electrodes being disposed along the plurality of spines from the first end to a point along the spine approximately at a distal end of the basket when in the expanded configuration.
Clause 10: The medical probe of clause 8 or clause 9, the plurality of electrodes being configured to detect electrophysiological signals.
Clause 11: The medical probe of any of clauses 8-10, the plurality of electrodes being configured to deliver ablative energy to tissue.
Clause 12: The medical probe of clause 1, each spine of the plurality of spines configured to turn outwardly away from the longitudinal axis such that the second end is disposed near the first end and each spine bows radially outward from the longitudinal axis when deployed from an insertion sheath.
Clause 13: The medical probe of clause 12, the plurality of electrodes being disposed along the plurality of spines from the second end to a point along the spine approximately at a distal end of the basket when in the expanded configuration.
Clause 14: The medical probe of clause 12 or clause 13, the plurality of electrodes being configured to detect electrophysiological signals.
Clause 15: The medical probe of any of clauses 12-14, the plurality of electrodes being configured to deliver ablative energy to tissue.
Clause 16: The medical probe of any of clauses 12-15, each spine of the plurality of spines comprising an anchor disposed at the second end, each anchor configured to attach to the insertion sheath when in the expanded position.
Clause 17: The medical probe of any of the preceding clauses, each spine of the plurality of spines comprising a shape memory material.
Clause 18: The medical probe of any of the preceding clauses, the shape memory material comprising nitinol.
Clause 19: A medical probe comprising: a tube comprising a proximal end and a distal end, the tube extending along a longitudinal axis; a plurality of spines disposed at a distal end of the tube, each spine of the plurality of spines comprising a first end and a second end each being fixed in relation to the tube, each spine of the plurality of spines configured to transition between a collapsed configuration and an expanded configuration, the plurality of spines forming a basket when in the expanded configuration by folding approximately at a midpoint of each spine and bowing radially outward from the longitudinal axis; and a plurality of electrodes attached to the plurality of spines
Clause 20: The medical probe of clause 19, the midpoint of each spine being configured to converge near a distal end of the basket when in the expanded configuration.
Clause 21: The medical probe of clause 20, wherein the plurality of spines comprises three spines.
Clause 22: The medical probe of any of clauses 19-21, the plurality of electrodes being configured to detect electrophysiological signals.
Clause 23: The medical probe of any of clauses 19-22, the plurality of electrodes being configured to deliver ablative energy to tissue.
Clause 24: A medical probe comprising: a tube comprising a proximal end and a distal end, the tube extending along a longitudinal axis; a single spine disposed at a distal end of the tube and comprising a first end and a second end each fixed in relation to the tube, the spine configured to transition between a collapsed configuration and an expanded configuration, the spine forming a basket comprising multiple lobes when in the expanded configuration by forming a plurality of bends, the multiple lobes bowing radially outward from the longitudinal axis; and a plurality of electrodes attached to the spine.
Clause 25: The medical probe of clause 24, wherein the basket comprises at least three lobes.
Clause 26: The medical probe of clause 24 or clause 25, the plurality of electrodes being configured to detect electrophysiological signals.
Clause 27: The medical probe of any of clauses 24-26, the plurality of electrodes being configured to deliver ablative energy to tissue.

The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

## Claims

1. A medical probe comprising:
a tube comprising a proximal end and a distal end, the tube extending along a longitudinal axis;
a plurality of spines disposed at the distal end of the tube, each spine of the plurality of spines comprising a first end that is fixed in relation to the tube and a second end that is unattached in relation to the tube, each spine of the plurality of spines configured to transition between a collapsed configuration and an expanded configuration, the plurality of spines forming a basket when in the expanded configuration; and
a plurality of electrodes attached to the plurality of spines.

2. The medical probe of claim 1, each spine of the plurality of spines configured to turn inwardly at a distal end and bow radially outward from the longitudinal axis when deployed from an insertion sheath.

3. The medical probe of claim 2 further comprising a spacer disposed between the plurality of spines so that the plurality of spines are spaced apart from each other, optionally the spacer being connected to a pull wire, the pull wire being configured to cause the spacer to move proximally thereby causing the plurality of spines to be spaced apart from each other.

4. The medical probe of any preceding claim, the plurality of electrodes being disposed along each spine of the plurality of spines from the first end to the second end, and/or being configured to detect electrophysiological signals, and/or being configured to deliver ablative energy to tissue.

5. The medical probe of claim 1, each spine of the plurality of spines configured to turn inwardly toward the longitudinal axis such that the second end is disposed near the first end and each spine bows radially outward from the longitudinal axis when deployed from an insertion sheath.

6. The medical probe of claim 5, the plurality of electrodes being disposed along the plurality of spines from the first end to a point along the spine approximately at a distal end of the basket when in the expanded configuration.

7. The medical probe of claim 5 or claim 6, the plurality of electrodes being configured to detect electrophysiological signals and/or to deliver ablative energy to tissue.

8. The medical probe of claim 1, each spine of the plurality of spines configured to turn outwardly away from the longitudinal axis such that the second end is disposed near the first end and each spine bows radially outward from the longitudinal axis when deployed from an insertion sheath.

9. The medical probe of claim 8, the plurality of electrodes being disposed along the plurality of spines from the second end to a point along the spine approximately at a distal end of the basket when in the expanded configuration.

10. The medical probe of claim 8 or claim 9, the plurality of electrodes being configured to detect electrophysiological signals and/or to deliver ablative energy to tissue.

11. The medical probe of any of claims 8 to 10, each spine of the plurality of spines comprising an anchor disposed at the second end, each anchor configured to attach to the insertion sheath when in the expanded position.

12. The medical probe of any preceding claim, each spine of the plurality of spines comprising a shape memory material.

13. A medical probe comprising:
a tube comprising a proximal end and a distal end, the tube extending along a longitudinal axis;
a plurality of spines disposed at a distal end of the tube, each spine of the plurality of spines comprising a first end and a second end each being fixed in relation to the tube, each spine of the plurality of spines configured to transition between a collapsed configuration and an expanded configuration, the plurality of spines forming a basket when in the expanded configuration by folding approximately at a midpoint of each spine and bowing radially outward from the longitudinal axis; and
a plurality of electrodes attached to the plurality of spines.

14. The medical probe of claim 13, the midpoint of each spine being configured to converge near a distal end of the basket when in the expanded configuration.

15. A medical probe comprising:
a tube comprising a proximal end and a distal end, the tube extending along a longitudinal axis;
a single spine disposed at a distal end of the tube and comprising a first end and a second end each fixed in relation to the tube, the spine configured to transition between a collapsed configuration and an expanded configuration, the spine forming a basket comprising multiple lobes when in the expanded configuration by forming a plurality of bends, the multiple lobes bowing radially outward from the longitudinal axis; and
a plurality of electrodes attached to the spine.
